(19) Europäisches Patentamt
European Patent Office
Office européen des brevets

(11) EP 3 773 926 B1

(12) **EUROPEAN PATENT SPECIFICATION**

(45) Date of publication and mention of the grant of the patent:
**06.10.2021 Bulletin 2021/40**

(21) Application number: **19740594.7**

(22) Date of filing: **23.07.2019**

(51) Int Cl.:
*A61Q 11/00* (2006.01) *A61K 8/39* (2006.01)
*A61K 8/49* (2006.01) *A61K 8/86* (2006.01)

(86) International application number:
**PCT/EP2019/069783**

(87) International publication number:
**WO 2020/035269 (20.02.2020 Gazette 2020/08)**

(54) **ORAL CARE COMPOSITION**

MUNDPFLEGEZUSAMMENSETZUNG

COMPOSITION D'HYGIÈNE BUCCALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.08.2018 PCT/CN2018/100957**
**28.09.2018 EP 18197616**

(43) Date of publication of application:
**17.02.2021 Bulletin 2021/07**

(73) Proprietors:
- **Unilever Global IP Limited**
**Wirral, CH62 AZD (GB)**
Designated Contracting States:
**CY DE GB IE IT MT**
- **Unilever IP Holdings B.V.**
**3013 AL Rotterdam (NL)**
Designated Contracting States:
**AL AT BE BG CH CZ DK EE ES FI FR GR HR HU IS LI LT LU LV MC MK NL NO PL PT RO RS SE SI SK SM TR**

(72) Inventors:
- **ANACHKOV, Svetoslav Emilov**
**1164 Sofia (BG)**
- **JIN, Huajin**
**Shanghai, Changning District 200335 (CN)**
- **KRALCHEVSKY, Peter Atanassov**
**1164 Sofia (BG)**
- **LI, Yajuan**
**Shanghai, Xuhui District (CN)**

(74) Representative: **Tansley, Sally Elizabeth et al**
**Unilever N.V.**
**Unilever Patent Group**
**Bronland 14**
**6708 WH Wageningen (NL)**

(56) References cited:
**US-A1- 2014 322 140 US-A1- 2016 331 663**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 773 926 B1

Printed by Jouve, 75001 PARIS (FR)

## Description

### Field of the Invention

The present invention is concerned with oral care compositions such as toothpastes, powders, gums, serums, mouthwashes and the like. More particularly, the present invention is concerned with oral care compositions comprising a non-ionic surfactant and a pigment. The invention is also concerned with the use of such compositions for whitening the teeth of an individual.

### Background of the Invention

The enamel layer of the tooth is naturally an opaque white or slightly off-white colour. The colour of teeth is influenced by a combination of their intrinsic colour and the presence of any extrinsic stains that may form on the tooth surface. Many products we consume have a negative impact on our teeth and mouth. Many substances can stain or reduce the whiteness of one's teeth, in particular, certain foods, tobacco products, and fluids such as tea and coffee. These staining and discolouring substances are often able to permeate the enamel layer. This problem occurs gradually over many years, but imparts a noticeable discoloration of the enamel of one's teeth.

Consumers have always had a strong desire for whiter teeth and many individuals are dissatisfied with their current teeth colour. This desire for whiter teeth has given rise to a growing trend in the increased use of tooth whitening products which range from toothpastes to mouthwashes and chewing gums. The whitening effect can be produced by chemically altering or removing the stain and/or changing the visual perception of the color of the teeth. Many current whitening toothpastes comprise abrasives and chemical components to clean and whiten teeth. Abrasive ingredients used to remove extrinsic stains may cause damage to the teeth and gums if overused.

It is known in the literature that the visual perception of a white substance can be altered through the deposition of an optical brightener, blue pigment or blue dye. Blue Covarine is a phthalocyanine blue pigment present in the toothpaste named white Now® and there have been attempts to enhance the deposition of this pigment on teeth for instant whitening benefits in oral care products.

Surfactants are commonly included in oral care products like toothpastes to help cleaning and cause foaming during use of the product. Foaming is experienced by the user that the product is effective in cleaning. But the use of surfactants in oral care products may prevent effective deposition of pigments on tooth surfaces due to their cleaning performance.

WO 2016/099544 A1 (Colgate-Palmolive Company) discloses a tooth whitening oral care composition made by combining ingredients comprising flakes of a water soluble whitening film, a dye with a hue angle in the CIELAB system ranging from 200 to 320 degrees and an orally acceptable carrier vehicle.

EP 1 935 395 A1 (Unilever) discloses from 0.01 to 0.3% by weight of a pigment having a hue angle, h, in the CIELAB system of from 220 to 320 degrees, characterized in that the composition further comprises a soluble deposition aid for said pigment.

WO 2012/123241 A2 (Unilever) discloses an oral care composition suitable for delivering a temporary whitening effect to the surface of teeth, the composition comprising: a continuous phase comprising water or polyhydric alcohol or a mixture thereof; a particulate tooth surface whitening agent which is dispersed in the continuous phase, and a deposition aid for the particulate tooth surfaces whitening agent; characterised in that the deposition aid is a poly-(sulphonic acid) polymer. The particulate tooth whitening agent is a phthalocyanine blue pigment.

US 2016/331663 A1 and US2014/322140 A1 (Colgate-Palmolive Company) disclose oral care compositions based on blue pigments.

There is continuing need to develop an oral care composition which maintains good deposition of pigments in the presence of surfactants to deliver instant tooth whitening benefit.

### Test and definitions

#### Dentifrice

"Dentifrice" for the purposes of the present invention means a paste, powder, liquid, gum or other preparation for cleaning the teeth or other surfaces in the oral cavity.

#### Toothpaste

"Toothpaste" for the purpose of the present invention means a paste or gel dentifrice for use with a toothbrush. Especially preferred are toothpastes suitable for cleaning teeth by brushing for about two minutes.

Mouth Wash

"Mouth wash" for the purpose of the present invention means liquid dentifrice for use in rinsing the mouth. Especially preferred are mouth washes suitable for rinsing the mouth by swishing and/or gargling for about half a minute before expectorating.

pH

pH is quoted at atmospheric pressure and a temperature of 25°C. When referring to the pH of an oral care composition, this means the pH measured when 5 parts by weight of the composition is uniformly dispersed and/or dissolved in 20 parts by weight pure water at 25°C. In particular, the pH may be measured by manually mixing 5 g oral care composition with 20 mL water for 30 s, then immediately testing the pH with indicator or a pH meter.

Solubility

"Soluble" and "insoluble" for the purpose of the present invention, refers to the solubility of a source in water at 25°C and atmospheric pressure. "Soluble" means a source that dissolves in water to give a solution with a concentration of at least 0.1 moles per litre. "Insoluble" means a source that dissolves in water to give a solution with a concentration of less than 0.001 moles per litre. "Slightly soluble", therefore, is defined to mean a source that dissolves in water to give a solution with a concentration of greater than 0.001 moles per litre and less than 0.1 moles per litre.

Polymers

"Polymers" for the purpose of the present invention, refers to polymeric material having large molecules composed of many repeated subunits. The polymer according to the present invention has a molecular weight of at least 10,000 g/mol, preferably from 10,000 to 5,000,000 g/mol. "Molecular weight" for the purpose of the present invention, refers to the weight average molecular mass of a given polymer. The weight average molecular weight of polymers is determined by gel permeation chromatography against a polyethylene glycol standard.

Viscosity

Viscosity of a toothpaste is the value taken at room temperature (25°C) with a Brookfield Viscometer, Spindle No.93/94 and at a speed of 5 rpm for 1 minute. Values are quoted in centipoises (cP=mPa.s) unless otherwise specified.

Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final oral care composition, unless otherwise specified. It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

Where a feature is disclosed with respect to a particular aspect of the invention (for example a composition of the invention), such disclosure is also to be considered to apply to any other aspect of the invention (for example a method of the invention) *mutatis mutandis.*

**Summary of the Invention**

In a first aspect, the present invention is directed to an oral care composition comprising:

a) a non-ionic surfactant comprising one or more carbon-carbon double bond;
b) a pigment having a hue angle, h, in the CIELAB system of from 220 to 320 degrees;
c) a physiologically acceptable carrier;

wherein the non-ionic surfactant and the pigment are present in a weight ratio of from 1:1 to 20:1; and
wherein the composition does not comprise anionic surfactants, amphoteric surfactants and polymers selected from carbomers, guar gums, guar hydroxypropyltrimmonium chloride, poloxamers, copolymers of methyl vinyl ether and

maleic anhydride, and mixtures thereof.

In a second aspect, the present invention is directed to an oral care composition comprising:

a) a non-ionic surfactant comprising one or more carbon-carbon double bond;
b) a pigment having a hue angle, h, in the CIELAB system of from 220 to 320 degrees;
c) a physiologically acceptable carrier;

wherein the non-ionic surfactant and the pigment are present in a weight ratio of from 1:1 to 20:1;

wherein the composition comprises other surfactants in addition to the non-ionic surfactant comprising one or more carbon-carbon double bond, and the level of other surfactants is less than 0.01% by weight of the composition; and

wherein the composition comprises polymers selected from carbomers, guar gums, guar hydroxypropyltrimmonium chloride, poloxamers, copolymers of methyl vinyl ether and maleic anhydride, and mixtures thereof and the level of polymers is less than 0.1% by weight of the composition.

In a third aspect, the present invention is directed to a method of whitening teeth of an individual comprising the step of applying the oral care composition of any embodiment of the first or second aspect to at least one surface of the teeth of the individual.

All other aspects of the present invention will more readily become apparent upon considering the detailed description and examples which follow.

**Detailed Description of the Invention**

The non-ionic surfactant suitable for use in the composition of the invention comprises one or more carbon-carbon double bond. The carbon-carbon double bond comprises aromatic, non-aromatic double bond or a mixture thereof.

Preferred non-ionic surfactants comprise polyoxyethylene sorbitan alkyl esters, alkyl phenol ethoxylates or a mixture thereof. Polyoxyethylene sorbitan alkyl ester is generally known as the Tween™ class, which has the general structure (I) as given below:

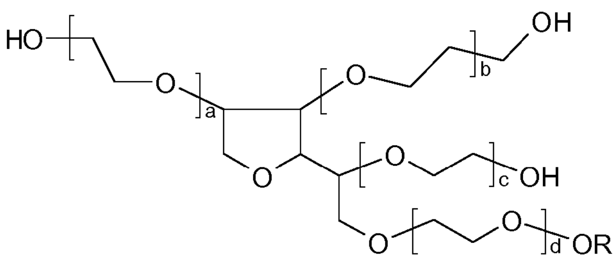

(I)

where R is $C_{12-18}$ carbon chain, and the sum of a, b, c and d is 20.

Polyoxyethylene (20) sorbitan monooleate (Tween 80) which comprises unsaturated oleic chain is particularly preferred.

Alkyl phenol ethoxylates are generally known as the Triton™ class, which has the general structure (II) as given below:

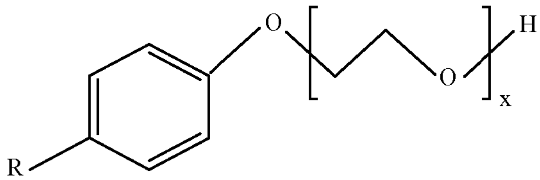

(II)

Where R is $C_8$ carbon chain, and x is an integer of from 7 to 70.

Preferred non-ionic surfactant of the alkyl phenol ethoxylates include Triton X-100, Triton-102, Triton-114, Triton-305, Triton X-405, Triton-705 or a mixture thereof.

Polyoxyethylene (10) tert-octylphenyl ether (Triton X-100) or polyoxyethylene (40) tert-octylphenyl ether (Triton X-405) is particularly preferred.

Most preferably, the non-ionic surfactant suitable for use in the present invention is polyoxyethylene (20) sorbitan monooleate, polyoxyethylene (10) tert-octylphenyl ether, polyoxyethylene (40) tert-octylphenyl ether or a mixture thereof.

The oral care composition of the present invention typically comprises from 0.01 to 10% by weight of the non-ionic surfactant which comprises one or more carbon-carbon double bond, more preferably from 0.02 to 5%, and most preferably from 0.05 to 3%, based on total weight of the oral care composition.

The pigment according to the invention is a shade/material which is insoluble in the relevant medium, at the relevant temperature. This is in contrast to dyes which are soluble. The term "relevant medium", as used herein, refers to human saliva, the liquid medium in which the composition is used, at the temperature of the oral cavity during brushing of the teeth, i.e. up to 37°C. The relevant medium may also be water and the relevant temperature to be 25°C. The only limitation with respect to the pigment is that the same is suitable for use in the mouth.

The pigment has a hue angle, h, in the CIELAB system of from 220 to 320 degrees, more preferably from 250 to 290 degrees. A detailed description of hue angels may be found on p57 of Colour Chemistry 3rd edition by H. Zollinger published by Wiley-VCH. Preferably the pigment is violet or blue, more preferably the pigment is selected from one or more of those listed in the Colour Index International as pigment blue 1 through to pigment 83 and pigment violet 1 through to pigment violet 56. Other suitable pigments are pigment ultramarine blue and ultramarine violet. While the preferred pigment is blue or violet, the same effect may be achieved through mixing pigments outside of this hue angle range. For example, such a hue angle may also be obtained by mixing a red and green-blue pigment to yield a blue or violet shaded pigment.

It is particularly preferred that the pigment is a blue pigment. Some examples of blue pigments suitable for use in this invention include inorganic blue pigments such as iron blue (C.I.77510) and ultramarine blue (C.I.77007). A preferred class of blue pigments suitable for use in the invention are organic blue pigments such as phthalocyanine blue pigments. Phthalocyanines are organometallic compounds containing four symmetrically arranged isoindole rings connected in a 16-membered ring linking with alternate carbon and nitrogen atoms. Most phthalocyanines contains a central, coordinated metal ion such as copper. Copper phthalocyanines have the basis structure:

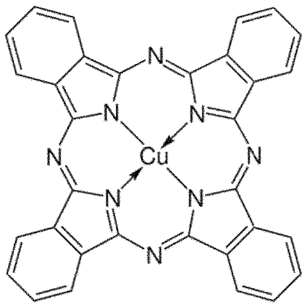

Phthalocyanine blue pigments exhibit more than one crystal modification, which differ in terms of coloristics. Methods have been developed to phase-stabilise the phthalocyanine pigment molecule in order to prevent conversion to a different crystal modification. An example is minor chemical modification of the molecule, for instance partial chlorination. Methods have also been developed to stabilise the phthalocyanine pigment molecule against flocculation during pigment application. An example is admixture of other agents to the molecule, such as surface active additives to the pigment molecule. The following pigments are illustrative phthalocyanine blue pigments preferably included in the composition according to the invention:

| C.I. Generic Name | C.I. Constitution Number | Crystal modification; Type | Number of halogen atoms |
|---|---|---|---|
| Pigment Blue 15 | 74160 | alpha | -- |
| Pigment Blue 15:1 | 74160 | alpha; phase stabilised | 0.5-1 Cl |
| Pigment Blue 15:2 | 74160 | alpha; phase & flocculation stabilised | 0.5-1 Cl |
| Pigment Blue 15:3 | 74160 | beta | -- |
| Pigment Blue 15:4 | 74160 | beta; flocculation stabilised | -- |
| Pigment Blue 15:6 | 74160 | epsilon | -- |
| Pigment Blue 16 | 74100 | gamma; metal-free | -- |

It is especially preferred that the pigment is phthalocyanine blue pigment selected from alpha copper phthalocyanines Pigment Blue 15, Pigment Blue 15:1, Pigment 15:2 and mixtures thereof. Most preferably the pigment is Pigment Blue 15:1. A commercially available example is Cosmenyl Blue A4R from Clariant.

The pigment suitable for use in this invention may also be mixtures of any of the above described materials.

Typically, the pigment is present in oral care composition of the invention in an amount from 0.001 to 1% by weight of the oral care composition, more preferably from 0.01 to 0.5%, and most preferably from 0.01 to 0.2%, based on total weight of the oral care composition.

The oral care composition of the present invention is found to maintain good deposition efficacy of pigments in the presence of certain non-ionic surfactants. Without wishing to be bound by theory, the present inventors believe that in the presence of non-ionic surfactants, the pigment and the tooth surface are sterically stabilized. While anionic surfactants such as sodium lauryl sulfate bring negative charges to both the pigment and the tooth surface. As a result, the negatively charged pigment is repelled from the tooth surface. While the electrostatic repulsion is relatively long-ranged, the steric repulsion is substantial at distances that are close to the headgroup length. In addition, the non-ionic surfactants comprising one or more carbon-carbon double bond can interact via $\pi$-$\pi$ interactions ($\pi$-stacking) with the aromatic rings in the pigment such as phthalocyanine blue pigment. Such interactions are expected to be beneficial for the deposition of pigments.

The weight ratio of the non-ionic surfactant comprising one or more carbon-carbon double bond and the pigment are present in a weight ratio of from 1:1 to 20:1, preferably from 1:1 to 15:1, more preferably from 2:1 to 15:1, and most preferably from 2:1 to 12:1.

It is preferred if the oral care composition of the present invention does not comprise anionic surfactants and amphoteric surfactants. More preferably, the oral care composition does not comprise other surfactants in addition to the non-ionic surfactant comprising one or more carbon-carbon double bond which is included in the composition. If other surfactants are present, it is preferred if the non-ionic surfactant comprising one or more carbon-carbon double bond is at least 75% by weight of the surfactants in the composition, more preferably from 80 to 100% and most preferably from 95 to 100% based on total weight of the surfactants in the composition.

If the oral care composition comprises other surfactants in addition to the non-ionic surfactant comprising one or more carbon-carbon double bond, it is preferred if they are present in a level less than 0.01% by weight of the composition, more preferably less than 0.001%. Typical anionic surfactants include, for example, sodium, magnesium, ammonium or ethanolamine salts of $C_8$ to $C_{18}$ alkyl sulphates (for example sodium lauryl sulfate), ethoxylated alkyl sulfate anionic surfactant (such as sodium lauryl ether sulfate), $C_8$ to $C_{18}$ alkyl sulphosuccinates (for example dioctyl sodium sulphosuccinate), $C_8$ to $C_{18}$ alkyl sulphoacetates (such as sodium lauryl sulphoacetate), $C_8$ to $C_{18}$ alkyl sarcosinates (such as sodium lauryl sarcosinate), $C_8$ to $C_{18}$ alkyl phosphates (which can optionally comprise up to 10 ethylene oxide and/or propylene oxide units) and sulphated monoglycerides, preferably the anionic surfactant is sodium lauryl sulfate. Typical amphoteric surfactants include, for example, alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms, preferably the amphoteric surfactant is cocamidopropyl betaine.

Additionally, it is also preferred that the composition of the present invention does not comprise polymers selected from carbomers, guar gums, guar hydroxypropyltrimmonium chloride, poloxamers, copolymers of methyl vinyl ether and maleic anhydride, and mixtures thereof. More preferably, the composition does not comprise polymers selected from anionic, non-ionic and cationic polymers.

When the oral care composition comprises polymers selected from carbomers, guar gums, guar hydroxypropyltrimmonium chloride, poloxamers, copolymers of methyl vinyl ether and maleic anhydride, and mixtures thereof, it is preferred they are presented in a level less than 0.1%, more preferably less than 0.01%, and most preferably less than 0.001% by weight of the composition.

Preferably, the oral care composition has a pH from 4.0 to 10.0, more preferably from 5.0 to 9.0, and most preferably from 5.5 to 8.0.

The composition of the present invention is an oral care composition and comprises a physiologically acceptable carrier. Water is the most common carrier for this invention. The carrier may further comprise at least thickener, humectant or a combination thereof.

Thickener may be used in this invention and is limited only to the extent that the same may be added to a composition suitable for use in the mouth. Illustrative examples of the types of thickeners that may be used in this invention include silica based thickeners including silica aerogels (e.g. sorbosil TC15), magnesium aluminum silicate (e.g., Veegum), and clays (e. g. Bentonite).

Thickener typically makes up from 0.1 to about 15%, more preferably from 1 to 12%, and most preferably, from 5 to 10% by weight of the oral care composition, based on total weight of the composition.

The oral care composition of the invention is preferably a toothpastes or gel. When the oral care composition is a toothpaste or gel, the same typically has a viscosity from about 30,000 to 180,000 centipoise, and preferably, from

60,000 to 170,000 centipoise, and most preferably, from 65,000 to 165,000 centipoise.

Suitable humectants are preferably used in the oral care composition of the present invention and they include, for example, glycerin, sorbitol, propylene glycol, dipropylene glycol, diglycerol, triacetin, mineral oil, polyethylene glycol (preferably, PEG-400), alkane diols like butane diol and hexanediol, ethanol, pentylene glycol, or a mixture thereof. Glycerin, polyethylene glycol, sorbitol or mixtures thereof are the preferred humectants.

The humectant may be present in the range of from 10 to 90% by weight of the oral care composition. More preferably, the carrier humectant makes up from 25 to 80%, and most preferably, from 30 to 70% by weight of the composition, based on total weight of the composition.

The oral care composition of the present invention may contain a variety of other ingredients which are common in the art to enhance physical properties and performance, in addition to pigments which are included in the composition. These ingredients include anti-microbial agents, anti-inflammatory agents, anti-caries agents, plaque buffers, fluoride sources, vitamins, plant extracts, desensitizing agents, anti-calculus agents, biomolecules, flavors, proteinaceous materials, preservatives, opacifying agents, pH-adjusting agents, sweetening agents, particulate abrasive materials, buffers and salts to buffer the pH and ionic strength of the compositions, and mixtures thereof. Such ingredients typically and collectively make-up less than 20% by weight of the composition, and preferably, from 0.0 to 15% by weight, and most preferably, from 0.01 to 12% by weight of the composition.

The oral care composition of this invention can be used in a method of whitening the teeth of an individual comprising applying the composition to at least one surface of the teeth of the individual. The oral care composition of this invention may additionally or alternatively be for use as a medicament and/or used in the manufacture of a medicament for providing an oral care benefit as described herein, such as for whitening the teeth of an individual. Alternatively and preferably, the use is non-therapeutic.

Typically, the composition will be packaged. In toothpaste or gel form, the composition may be packaged in a conventional plastic laminate, metal tube or a single compartment dispenser. The same may be applied to dental surfaces by any physical means, such as a toothbrush, fingertip or by an applicator directly to the sensitive area. In liquid mouthwash form the composition may be packaged in a bottle, sachet or other convenient container.

The composition can be effective even when used in an individual's daily oral hygiene routine. For example, the composition may be brushed onto the teeth and/or be rinsed around the inside of the mouth of the individual. The composition may, for example, be contacted with the teeth for a time period of one second to 20 hours. More preferably from 1 s to 10 hours, more preferably still from 10 s to 1 hour and most preferably from 30 s to 5 minutes. The composition may be used daily, for example for use by an individual once, twice or three times per day.

The following examples are provided to facilitate an understanding of the present invention. The examples are not provided to limit the scope of the claims.

## Examples

### Example 1

This example demonstrated the deposition of pigments by using different surfactants. All ingredients are expressed by weight percent of the total formulation, and as level of active ingredient.

**TABLE 1**

| Ingredients | Samples | | | | | | | | |
|---|---|---|---|---|---|---|---|---|---|
| | 1 | 2 | 3 | 4 | 5 | 6 | 7 | 8 | 9 |
| Triton X-100 | -- | 0.30 | -- | -- | -- | -- | -- | -- | -- |
| Tween-80 | -- | -- | 0.30 | -- | 0.18 | 0.06 | 0.24 | 0.12 | -- |
| Triton X-405 | -- | -- | -- | 0.30 | -- | -- | -- | -- | -- |
| SLS | -- | -- | -- | -- | 0.12 | 0.24 | 0.06 | 0.18 | 0.30 |
| Blue Covarine[a] (37.8%) | 0.075 | 0.075 | 0.075 | 0.075 | 0.075 | 0.075 | 0.075 | 0.075 | 0.075 |
| Water | To 100 | To 100 | To 100 | To 100 | To 100 | To 100 | To 100 | To 100 | To 100 |
| a. Dispersion of C.I. 74160 (pigment blue 15:1) in water/glycerol under the trade name Cosmenyl Blue A4R from Clariant. | | | | | | | | | |

*Methods*

Regenerated hydroxyapatite (HAP) discs were used in this test, which were prepared following the procedure: the surface layer of each HAP disc was removed using sandpaper P220 with grit size of ca. 68 μm. After that, the HAP discs were cleaned via sonication for 30 mins in distilled water, followed by sonication for 30 mins in ethanol. Then they were air-dried.

To evaluate the deposition of pigments, the following in vitro test was performed. Each HAP disc was soaked in de-ionized (DI) water for 1 hour. After soaking, the HAP disc was immersed directly in a test sample for 5 mins. Then the disc was placed in DI water for 5 mins to wash away any excess pigments that was weakly bound to the disc surface (analogous to mouth rinsing). Finally, the HAP disc was air-dried at room temperature and kept for image analysis.

The image analysis was carried out using the ImageJ software, whereas the image preprocessing was done using Wolfram Mathematica version 11.1. All HAP discs were placed in order on a printed grid (table) and then a photograph of all discs was taken by a digital camera. For the sake of comparison, all discs should fit in a single photograph. The photograph was then converted to a 32-bit grayscale image. The mean-grey-value (MGV) measurement was chosen in ImageJ. The relative dye intensity RDI was evaluated as follows:

$$RDI = 10\frac{MGV_{max} - MGV}{MGV_{max} - MGV_{min}}$$

MGV is the mean grey value. $MGV_{min}$ and $MGV_{max}$ are the minimal and maximal MGV (varying for each image).

*Results*

The results are summarized in Table 2 (error represents standard deviation for duplicate measurements). The higher the RDI value the more deposition of the pigment.

**TABLE 2**

| | **Samples** | | | | | | | | |
|---|---|---|---|---|---|---|---|---|---|
| | **1** | **2** | **3** | **4** | **5** | **6** | **7** | **8** | **9** |
| RDI | 10.0±0.9 | 8.5±1.0 | 4.6±1.0 | 4.0±0.9 | 0.7±1.0 | 0.1±1.0 | 0.1±1.0 | 0.0±1.0 | 0.0±1.0 |

Samples 2 to 4 comprising non-ionic surfactants with carbon-carbon double bond had much higher RDI values compared to Sample 9 which comprised only SLS (sodium lauryl sulfate). Besides, Samples 5 to 8 comprising a combination of Tween-80 and sodium lauryl sulfate also showed inferior performance to Samples 2 to 4 which comprised only non-ionic surfactants.

Example 2

This example demonstrated the effect of amphoteric surfactants on the deposition of pigments. All ingredients are expressed by weight percent of the total formulation, and as level of active ingredient.

**TABLE 3**

| **Ingredients** | **Samples** | | | | | | |
|---|---|---|---|---|---|---|---|
| | **10** | **11** | **12** | **13** | **14** | **15** | **16** |
| Tween-80 | -- | 0.30 | 0.24 | 0.18 | -- | 0.06 | 0.12 |
| CAPB | -- | -- | 0.06 | 0.12 | 0.30 | 0.24 | 0.18 |
| Blue Covarine[a] (37.8%) | 0.075 | 0.075 | 0.075 | 0.075 | 0.075 | 0.075 | 0.075 |
| Water | To 100 | To 100 | To 100 | To 100 | To 100 | To 100 | To 100 |

*Methods*

Regenerated HAP discs were used in the test. The same protocol was used to evaluate the deposition of

pigments as described in Example 1.

*Results*

The results are summarized in Table 4 (error represents standard deviation for duplicate measurements).

**TABLE 4**

| | Samples | | | | | | |
|---|---|---|---|---|---|---|---|
| | **10** | **11** | **12** | **13** | **14** | **15** | **16** |
| RDI | 10.0±0.9 | 4.2±1.0 | 2.0±1.0 | 0.6±1.0 | 0.4±1.0 | 0.3±0.9 | 0.0±0.9 |

Samples 12 to 16 comprising additional CAPB (cocamidopropyl betaine) showed inferior performance to Sample 11 comprising only Tween-80.

Example 3

This example demonstrated the effect of polymers on the deposition of pigments. All ingredients are expressed by weight percent of the total formulation, and as level of active ingredient.

**TABLE 5**

| **Ingredients** | **Samples** | | | | | | | |
|---|---|---|---|---|---|---|---|---|
| | **17** | **18** | **19** | **20** | **21** | **22** | **23** | **24** |
| Triton X-100 | -- | 0.30 | 0.30 | 0.30 | 0.30 | 0.30 | 0.30 | 0.30 |
| Carbopol 2020[b] | -- | -- | 0.33 | -- | -- | -- | -- | -- |
| JaguarS[c] | -- | -- | -- | 0.33 | -- | -- | -- | -- |
| Poloxamer 407[d] | -- | -- | -- | -- | 0.33 | -- | -- | -- |
| Carbopol 971P[e] | -- | -- | -- | -- | -- | 0.33 | -- | -- |
| Gantrez[f] | -- | -- | -- | -- | -- | -- | 0.33 | -- |
| Jaguar C-500[g] | -- | -- | -- | -- | -- | -- | -- | 0.33 |
| Blue Covarine[a] (37.8%) | 0.075 | 0.075 | 0.075 | 0.075 | 0.075 | 0.075 | 0.075 | 0.075 |
| Water | To 100 | To 100 | To 100 | To 100 | To 100 | To 100 | To 100 | To 100 |

b. Commercial cross-linked polyacrylic acid copolymer under the trade name Carbopol ® ETD 2020 NF polymer from Lubrizol.
c. Commercial guar gum under the trade name Jaguar® S from Solvay.
d. Commercial available under the trade name Pluronic F127 from BASF.
e. Commercial available under the trade name Carbopol ® 971P NF from Lubrizol.
f. Commercial available under the trade name Gantrez® S-97 from Ashland.
g. Commercial available guar hydroxypropyltrimonium chloride under the trade name Jaguar® C-500 from Solvay.

*Methods*

Regenerated HAP discs were used in the test. The same protocol was used to evaluate the deposition of pigments as described in Example 1.

*Results*

The results are summarized in Table 6 (error represents standard deviation for duplicate measurements).

**TABLE 6**

| | Samples | | | | | | | |
|---|---|---|---|---|---|---|---|---|
| | **17** | **18** | **19** | **20** | **21** | **22** | **23** | **24** |
| RDI | 10.0±0.9 | 6.8±1.0 | 1.8±1.0 | 1.6±0.9 | 1.2±0.9 | 1.2±0.9 | 1.0±0.9 | 0.0±0.9 |

Samples 19 to 24 comprising additional polymers showed inferior performance to Sample 18 comprising only Triton X-100.

Example 4

This example demonstrated the weight ratio of non-ionic surfactant with carbon-carbon double bond to pigment can affect the deposition of pigments. All ingredients are expressed by weight percent of the total formulation, and as level of active ingredient.

**TABLE 7**

| Ingredients | Samples | |
|---|---|---|
| | **25** | **26** |
| Tween-60 | 0.281 | -- |
| Tween-80 | 0.019 | 0.3 |
| Blue Covarine[a] (37.8%) | 0.075 | 0.075 |
| Water | To 100 | To 100 |

*Methods*

To evaluate the deposition of pigments, the following in vitro test was performed. The colour difference expressed as the CIELAB delta E (ΔE) value was calculated based on L*, a*, b* values measured using DigiEye (VeriVide, England) with the following formula:

$$\Delta E = \sqrt{\Delta L^2 + \Delta a^2 + \Delta b^2}$$

Hydroxyapatite (HAP) discs were used as substrates. The baseline L*, a*, b* of the HAP discs was measured by DigiEye (VeriVide, England). Five HAP discs were used for each sample. The HAP discs were soaked in DI water for 2 hours. After soaking, the discs were immersed directly in test samples. After 2 hours, the HAP discs were taken out and rinsed with 40 mL DI water for 6 seconds. The residual water on discs were absorbed with a tissue. After this step the L*, a*, b* values of each HAP disc were re-measured based on which the delta E from baseline were calculated and statistically analyzed.

*Results*

The results are summarized in Table 8 (error represents standard deviation for duplicate measurements). The higher the delta E value the more deposition of pigments.

**TABLE 8**

| | Samples | |
|---|---|---|
| | **25** | **26** |
| Delta E | 49.85 ± 4.74 | 58.80 ± 2.12 |

Sample 26 comprising a higher weight ratio of non-ionic surfactant with carbon-carbon double bond to pigment showed much better deposition of pigments compared to sample 25.

## Claims

1. An oral care composition comprising:

a) a non-ionic surfactant comprising one or more carbon-carbon double bond;
b) a pigment having a hue angle, h, in the CIELAB system of from 220 to 320 degrees;
c) a physiologically acceptable carrier;

wherein the non-ionic surfactant and the pigment are present in a weight ratio of from 1:1 to 20:1; and wherein the composition does not comprise anionic surfactants, amphoteric surfactants and polymers selected from carbomers, guar gums, guar hydroxypropyltrimmonium chloride, poloxamers, copolymers of methyl vinyl ether and maleic anhydride, and mixtures thereof.

2. An oral care composition comprising:

a) a non-ionic surfactant comprising one or more carbon-carbon double bond;
b) a pigment having a hue angle, h, in the CIELAB system of from 220 to 320 degrees;
c) a physiologically acceptable carrier;
wherein the non-ionic surfactant and the pigment are present in a weight ratio of from 1:1 to 20:1;
wherein the composition comprises other surfactants in addition to the non-ionic surfactant comprising one or more carbon-carbon double bond, and the level of other surfactants is less than 0.01 % by weight of the composition; and
wherein the composition comprises polymers selected from carbomers, guar gums, guar hydroxypropyltrimmonium chloride, poloxamers, copolymers of methyl vinyl ether and maleic anhydride, and mixtures thereof and the level of polymers is less than 0.1 % by weight of the composition.

3. The oral care composition according to claim 1, wherein the composition does not comprise other surfactants in addition to the non-ionic surfactant comprising one or more carbon-carbon double bond.

4. The oral care composition according to any of the preceding claims, wherein the carbon-carbon double bond comprises aromatic, non-aromatic double bond or a mixture thereof.

5. The oral care composition according to any of the preceding claims, wherein the non-ionic surfactant comprises polyoxyethylene sorbitan alkyl esters, alkyl phenol ethoxylates or a mixture thereof.

6. The oral care composition according to claim 5, wherein the non-ionic surfactant is polyoxyethylene (20) sorbitan monooleate, polyoxyethylene (10) tert-octylphenyl ether, polyoxyethylene (40) tert-octylphenyl ether or a mixture thereof.

7. The oral care composition according to any of the preceding claims, wherein the composition comprises from 0.01 to 10% by weight of the non-ionic surfactant, preferably from 0.02 to 5%.

8. The oral care composition according to any of the preceding claims, wherein the pigment has a hue angle, h, in the CIELAB system of from 250 to 290 degrees.

9. The oral care composition according to any of the preceding claims, wherein the pigment is a blue pigment, preferably a phthalocyanine blue pigment.

10. The oral care composition according to claim 9, wherein the phthalocyanine blue pigment is selected from alpha copper phthalocyanines Pigment Blue 15, Pigment Blue 15:1, Pigment Blue 15:2 and mixtures thereof, preferably Pigment Blue 15:1.

11. The oral care composition according to any of the preceding claims, wherein the composition comprises from 0.001 to 1% by weight of the pigment, preferably from 0.01 to 0.5%.

12. The oral care composition according to any of the preceding claims, wherein the non-ionic surfactant and the pigment are present in a weight ratio of from 1:1 to 15:1, preferably from 2:1 to 15:1.

**13.** A non-therapeutic method of whitening teeth of an individual comprising the steps of applying the composition according to any of the preceding claims to at least one surface of the teeth of an individual.

## Patentansprüche

**1.** Mundpflegezusammensetzung, umfassend:

a) ein nichtionisches Tensid, umfassend eine oder mehrere Kohlenstoff-Kohlenstoff-Doppelbindungen;
b) ein Pigment mit einem Farbtonwinkel h im CIELAB-System von 220 bis 320 Grad;
c) einen physiologisch verträglichen Träger;

wobei das nichtionische Tensid und das Pigment in einem Gewichtsverhältnis von 1:1 bis 20:1 vorhanden sind; und wobei die Zusammensetzung keine anionischen Tenside, amphoteren Tenside und Polymere, ausgewählt aus Carbomeren, Guargummen, Guarhydroxypropyltrimmoniumchlorid, Poloxameren, Copolymeren von Methylvinylether und Maleinsäureanhydrid und Mischungen davon, umfasst.

**2.** Mundpflegezusammensetzung, umfassend:

a) ein nichtionisches Tensid, umfassend eine oder mehrere Kohlenstoff-Kohlenstoff-Doppelbindungen;
b) ein Pigment mit einem Farbtonwinkel h im CIELAB-System von 220 bis 320 Grad;
c) einen physiologisch verträglichen Träger;
wobei das nichtionische Tensid und das Pigment in einem Gewichtsverhältnis von 1:1 bis 20:1 vorhanden sind;
wobei die Zusammensetzung andere Tenside zusätzlich zu dem nichtionischen Tensid, das eine oder mehrere Kohlenstoff-Kohlenstoff-Doppelbindungen umfasst, umfasst und der Gehalt an anderen Tensiden weniger als 0,01 Gewichts-% der Zusammensetzung beträgt; und
wobei die Zusammensetzung Polymere umfasst, ausgewählt aus Carbomeren, Guargummen, Guarhydroxypropyltrimmoniumchlorid, Poloxameren, Copolymeren von Methylvinylether und Maleinsäureanhydrid und Mischungen davon, und der Anteil an Polymeren weniger als 0,1 Gewichts-% der Zusammensetzung beträgt.

**3.** Mundpflegezusammensetzung nach Anspruch 1, wobei die Zusammensetzung zusätzlich zu dem nichtionischen Tensid, das eine oder mehrere Kohlenstoff-Kohlenstoff-Doppelbindungen umfasst, keine anderen Tenside umfasst.

**4.** Mundpflegezusammensetzung nach irgendeinem der vorhergehenden Ansprüche, wobei die Kohlenstoff-Kohlenstoff-Doppelbindung eine aromatische, nichtaromatische Doppelbindung oder eine Mischung davon umfasst.

**5.** Mundpflegezusammensetzung nach irgendeinem der vorhergehenden Ansprüche, wobei das nichtionische Tensid Polyoxyethylensorbitanalkylester, Alkylphenolethoxylate oder eine Mischung davon umfasst.

**6.** Mundpflegezusammensetzung nach Anspruch 5, wobei das nichtionische Tensid Polyoxyethylen (20)-sorbitanmonooleat, Polyoxyethylen (10)-tert.-octylphenylether, Polyoxyethylen (40)-tert.-octylphenylether oder eine Mischung davon ist.

**7.** Mundpflegezusammensetzung nach irgendeinem der vorhergehenden Ansprüche, wobei die Zusammensetzung 0,01 bis 10 Gewichts-% des nichtionischen Tensids, bevorzugt 0,02 bis 5 %, umfasst.

**8.** Mundpflegezusammensetzung nach irgendeinem der vorhergehenden Ansprüche, wobei das Pigment einen Farbtonwinkel h im CIELAB-System von 250 bis 290 Grad aufweist.

**9.** Mundpflegezusammensetzung nach irgendeinem der vorhergehenden Ansprüche, wobei das Pigment ein blaues Pigment ist, vorzugsweise ein blaues Phthalocyanin-Pigment.

**10.** Mundpflegezusammensetzung nach Anspruch 9, wobei das blaue Phthalocyaninpigment ausgewählt ist aus alpha-Kupferphthalocyaninen Pigment Blue 15, Pigment Blue 15:1, Pigment Blue 15:2 und Mischungen davon, bevorzugt Pigment Blue 15:1.

**11.** Mundpflegezusammensetzung nach irgendeinem der vorhergehenden Ansprüche, wobei die Zusammensetzung 0,001 bis 1 Gewichts-% des Pigments, bevorzugt 0,01 bis 0,5%, umfasst.

**12.** Mundpflegezusammensetzung nach irgendeinem der vorhergehenden Ansprüche, wobei das nichtionische Tensid und das Pigment in einem Gewichtsverhältnis von 1:1 bis 15:1, bevorzugt 2:1 bis 15:1, vorhanden sind.

**13.** Nichttherapeutisches Verfahren zum Aufhellen der Zähne eines Individuums, umfassend die Schritte des Auftragens der Zusammensetzung nach irgendeinem der vorhergehenden Ansprüche auf mindestens eine Oberfläche der Zähne eines Individuums.

## Revendications

**1.** Composition pour le soin oral comprenant :

a) un tensioactif non-ionique comprenant une ou plusieurs doubles liaisons carbone-carbone ;
b) un pigment ayant un angle de teinte, h, dans le système CIELAB de 220 à 320 degrés ;
c) un support physiologiquement acceptable ;

dans laquelle le tensioactif non-ionique et le pigment sont présents dans un rapport de masse de 1:1 à 20:1 ; et dans laquelle la composition ne comprend pas de tensioactifs anioniques, tensioactifs amphotères et polymères choisis parmi des carbomères, gommes guar, chlorure de guar hydroxypropyltrimmonium, poloxamères, copolymères de méthylvinyléther et anhydride maléique, et mélanges de ceux-ci.

**2.** Composition pour le soin oral comprenant :

a) un tensioactif non-ionique comprenant une ou plusieurs doubles liaisons carbone-carbone ;
b) un pigment ayant un angle de teinte, h, dans le système CIELAB de 220 à 320 degrés ;
c) un support physiologiquement acceptable ;
dans laquelle le tensioactif non-ionique et le pigment sont présents dans un rapport de masse de 1:1 à 20:1 ;
dans laquelle la composition comprend d'autres tensioactifs en plus du tensioactif non-ionique comprenant une ou plusieurs doubles liaisons carbone-carbone, et la teneur d'autres tensio-actifs est inférieure à 0,01 % en masse de la composition ; et
dans laquelle la composition comprend des polymères choisis parmi des carbomères, gommes guar, chlorure de guar hydroxypropyltrimmonium, poloxamères, copolymères de méthylvinyléther et anhydride maléique, et mélanges de ceux-ci et la teneur en polymères est inférieure à 0,1 % en masse de la composition.

**3.** Composition pour le soin oral selon la revendication 1, dans laquelle la composition ne comprend pas d'autres tensioactifs en plus du tensioactif non-ionique comprenant une ou plusieurs doubles liaisons carbone-carbone.

**4.** Composition pour le soin oral selon l'une quelconque des revendications précédentes, dans laquelle la double liaison carbone-carbone comprend une double liaison aromatique, non-aromatique ou un mélange de celles-ci.

**5.** Composition pour le soin oral selon l'une quelconque des revendications précédentes, dans laquelle le tensioactif non-ionique comprend des esters alkyliques de polyoxyéthylène sorbitane, éthoxylates d'alkylphénol ou un mélange de ceux-ci.

**6.** Composition pour le soin oral selon la revendication 5, dans laquelle le tensioactif non-ionique est un monooléate de polyoxyéthylène (20) sorbitane, tert-octylphényléther de polyoxyéthylène (10), tert-octylphényléther de polyoxyéthylène (40) ou un mélange de ceux-ci.

**7.** Composition pour le soin oral selon l'une quelconque des revendications précédentes, dans laquelle la composition comprend de 0,01 à 10 % en masse du tensioactif non-ionique, de préférence de 0,02 à 5%.

**8.** Composition pour le soin oral selon l'une quelconque des revendications précédentes, dans laquelle le pigment présente un angle de teinte, h, dans le système CIELAB de 250 à 290 degrés.

**9.** Composition pour le soin oral selon l'une quelconque des revendications précédentes, dans laquelle le pigment est un pigment bleu, de préférence un pigment bleu de phthalocyanine.

**10.** Composition pour le soin oral selon la revendication 9, dans laquelle le pigment bleu de phthalocyanine est choisi

parmi des phthalocyanines de cuivre alpha Pigment Blue 15, Pigment Blue 15:1, Pigment Blue 15:2 et des mélanges de ceux-ci, de préférence Pigment Blue 15:1.

**11.** Composition pour le soin oral selon l'une quelconque des revendications précédentes, dans laquelle la composition comprend de 0,001 à 1 % en masse du pigment, de préférence de 0,01 à 0,5 %.

**12.** Composition pour le soin oral selon l'une quelconque des revendications précédentes, dans laquelle le tensioactif non-ionique et le pigment sont présents dans un rapport de masse de 1:1 à 15:1, de préférence de 2:1 à 15:1.

**13.** Procédé non-thérapeutique de blanchiment des dents d'un individu comprenant les étapes d'application de la composition selon l'une quelconque des revendications précédentes sur au moins une surface des dents d'un individu.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016099544 A1
- EP 1935395 A1
- WO 2012123241 A2
- US 2016331663 A1
- US 2014322140 A1


### Non-patent literature cited in the description


- **H. ZOLLINGER.** Colour Chemistry. Wiley-VCH, 57